# EUROPÄISCHE PATENTSCHRIFT

(11) **EP 1 348 417 B1**
(45) Veröffentlichungstag und Bekanntmachung des Hinweises auf die Patenterteilung: **07.09.2005**
(21) Anmeldenummer: 03007151.8
(22) Anmeldetag: 28.03.2003
(51) Int. Cl.: A61K 6/00

(54) **Quecksilberbindende Substanz für Dentalanwendungen**
Mercury complexing substance for dental use
Substance à usage dentaire pour complexer le mercure

(30) Priorität: 28.03.2002 DE 10213960
(43) Veröffentlichungstag der Anmeldung: 01.10.2003
(73) Patentinhaber: Hofmann, Andreas, 86420 Diedorf (DE); Wichnalek, Willi, Dr., 86420 Diedorf (DE); Wichnalek, Lydia, Dr., 86420 Diedorf (DE)
(72) Erfinder: Wichnalek, Willi, Dr., 86420 Diedorf (DE); Wichnalek, Lydia, Dr., 86420 Diedorf (DE)
(74) Vertreter: Vetter, Ewald Otto

(56) Entgegenhaltungen:
- WO-A-90/10433
- WO-A-95/23584
- WO-A-97/19670
- US-A- 5 242 305

## Beschreibung

Die Erfindung betrifft ein Zahneinlagenmaterial für Zahnkavitäten und ein Verfahren zu dessen Herstellung.

Die WO 97/19670 A offenbart eine Spülflüssigkeit zum Ausspülen der Mundhöhle zum Binden von Quecksilber, welches bei der mechanischen Behandlung von Amalgam, z. B. durch Bohrer oder andere abrasive Werkzeuge, frei wird.

Amalgame werden seit ungefähr 150 Jahren als Füllungsmaterial in der restaurativen Zahnheilkunde von menschlichen Zähnen eingesetzt. Die durchschnittliche Haltbarkeit einer Amalgamfüllung kann bis zu 10 und mehr Jahren betragen. In den letzten Jahren ist diese Verwendung immer mehr einer kritischen Betrachtung unterzogen worden. Der Hauptgrund hierfür ist das toxische und allergene Potential von Amalgam, insbesondere dessen Quecksilbergehalt. Wenn Quecksilber in den menschlichen Körper gelangt, kann dies Störungen im Stoffwechsel, besonders an den schwefelhaltigen Proteinen verursachen. Daher sind Amalgamfüllungen, auch wenn sie nur geringe Quecksilbermengen enthalten, ein Gesundheitsrisiko, da solche Amalgamfüllungen eine Art Dauerspender von Quecksilber für den menschlichen Organsismus darstellen. Trotz der geringen Mengen an dauernd freigesetztem Quecksilber sind eine ganze Reihe von Störungen und Krankheiten hierzu bekannt. Die wichtigsten Symptome von Amalgam-Intoxikationen und-Intoleranzen sind: gastrointestinale Störungen, Morbus Crohn, Myalgien, Migräne; psychische Veränderungen (Depressionen, Angstzustände etc.); Tremor; Lähmungserscheinungen; Sehbeschwerden; Zungenbrennen (verursacht durch Nervenschädigungen); Schluckbeschwerden; Beeinträchtigung des Geruchsinns; Fertilitätsstörungen; Tendenz zur Bildung von Aphten, Gingivitis; Parodontitis, Pilzinfektionen, Herpes im Mundbereich.

Von den Erfindern wurde überraschenderweise gefunden, dass auch nach der Entfernung einer Zahnfüllung aus Amalgam noch eine beträchtliche Restmenge Quecksilber in der ausgebohrten Zahnkavität und in der Zahnsubstanz vorhanden ist. Bei langjährigen Amalgamfüllungen dringt Quecksilber in die Zahnsubstanz ein, so dass diese mit Quecksilber imprägniert wird. Derzeit ist kein Mittel und kein Verfahren zur Entfernung von Quecksilber aus mit Quecksilber imprägnierter Zahnsubstanz bekannt. Der Zahnarzt entfernt zur Zeit mechanisch nur die sichtbaren Amalgame, aber nicht in die Zahnsubstanz imprägniertes Quecksilber und häufig auch nicht Quecksilber enthaltende Amalgampartikel in der Zahnkavität, aus welcher die Amalgamfüllung durch Ausbohren entfernt wurde. Bekannt ist lediglich bei akuten Quecksilbervergiftungen das Ausscheiden von Quecksilber über das Blutsystem oder den Gastro-Intestinal-Trakt des Menschen.

Durch die Erfindung soll die Aufgabe gelöst werden, die Quecksilberbelastung von Personen durch Zahnfüllungen aus Amalgam nach dem Entfernen des Amalgams aus der betreffenden Zahnkavität auf eine Weise zu verringern, welche einfach, preiswert und insbesondere für die betreffende Person nicht gesundheitsschädlich ist und keine für sie unangenehmen Verfahrenshandlungen erfordert.

Diese Aufgabe wird gemäß der Erfindung durch die Patentansprüche gelöst.

Demgemäß betrifft die Erfindung ein Zahneinlagenmaterial für Zahnkavitäten, welches eine Quecksilber-bindende Substanz enthält, für die Reduzierung der Quecksilberkonzentration in einer mit Quecksilber einer vorher entfernten Amalgamfüllung imprägnierten Zahnsubstanz, dadurch gekennzeichnet, dass die Quecksilber-bindende Substanz, in mikrokristalliner Zellulose, mindestens eines der folgenden Materialien enthält oder aus einem der folgenden Materialien besteht: Alkali-Thiosulfat, Natriumthiosulfat, Alkali-Selenit, Natriumselenit, Ethylendiamin-Tetraessigsäure (EDTA) und/oder DMPS.

Ferner betrifft die Erfindung ein Verfahren zur Herstellung von Zahneinlagenmaterial, welches eine Quecksilber-bindende Substanz enthält, für die Reduzierung der Quecksilberkonzentration in einer mit Quecksilber einer vorher entfernten Amalganfüllung imprägnierten Zahnsubstanz, gekennzeichnet durch die Verwendung von mindestens einem der folgenden Materialien als Quecksilberbindende Substanz in mikrokristalliner Zellulose: Alkali, Thiosulfat, Natriumthiosulfat, Alkali-Selenit, Natriumselenit, Ethylendiamin-Tetraessigsäure (EDTA) und/oder DMPS.

Die Erfindung kann zum Beispiel auf folgende Weise angewendet werden:
1. Entfernen der Amalgamfüllung, so dass keine sichtbaren Amalgamreste mehr in der Zahnkavität vorhanden sind;
2. Reinigung der Kavität von losen Anhaftungen,
3. Einbringung des Zahneinlagenmaterials in die Kavität, vorzugsweise auch Einbringen eines Farbstoffes in die Kavität, z. B. Erythrosin, zur Kontrolle der Dichtigkeit eines Kavitätenverschlusses;
4. Verschließen der Zahnkavität mit einem Kavitätenverschluss aus mineralischen und/oder polymeren Harzen, z. B. Bariumsulfat/Zinkoxid, Polyethylenfolien, Glas-Ionomeren; wobei die Zahnkavität als dicht verschlossen gilt, wenn sich kein gefärbter Rand zwischen Zahnwand und Kavitätenverschluss zeigt;
5. Inkubation über 7 oder 14 Tage je nach Alter oder Größe der früheren Amalgamfüllung. Danach Herausnehmen des Zahneinlagenmaterials aus der Zahnkavität und Beendigung der Sanierung oder optional Wechsel des Zahneinlagenmaterials gegen ein neues Zahneinlagenmaterial zur erneuten Inkubation im Bedarfsfall.

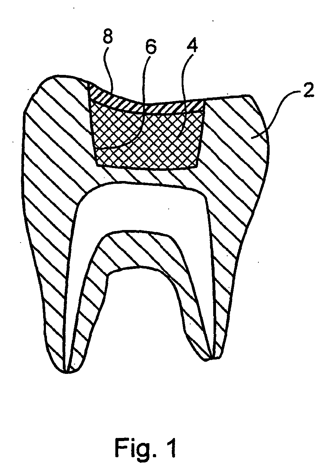

Die Erfindung wird im folgenden anhand einer Zeichnung als Beispiel beschrieben, welche mit Fig. 1 bezeichnet ist.

Fig. 1 zeigt einen Zahn 2 mit einer Zahneinlage 4 der vorstehend beschriebenen Art in einer Zahnkavität 6. Auf oder in der Zahneinlage 4 befindet sich Farbstoff der vorstehend beschriebenen Art in der Zahnkavität 6. Ein Verschluss 8 der Zahnkavität 6 befindet sich über der Zahneinlage 4 und über dem Farbstoff.

## Patentansprüche

1. Zahneinlagenmaterial für Zahnkavitäten, welches eine Quecksilber-bindende Substanz enthält, für die Reduzierung der Quecksilberkonzentration in einer mit Quecksilber einer vorher entfernten Amalgamfüllung imprägnierten Zahnsubstanz, **dadurch gekennzeichnet, dass** die Quecksilber-bindende Substanz, in mikrokristalliner Zellulose, mindestens eines der folgenden Materialien enthält oder aus einem der folgenden Materialien besteht: Alkali-Thiosulfat, Natriumthiosulfat, Alkali-Selenit, Natriumselenit, Ethylendiamin-Tetraessigsäure (EDTA) und/oder DMPS.

2. Verfahren zur Herstellung von Zahneinlagenmaterial, welches eine Quecksilber-bindende Substanz enthält, für die Reduzierung der Quecksilberkonzentration in einer mit Quecksilber einer vorher entfernten Amalganfüllung imprägnierten Zahnsubstanz, **gekennzeichnet durch** die Verwendung von mindestens einem der folgenden Materialien als Quecksilberbindende Substanz in mikrokristalliner Zellulose: Alkali-Thiosulfat, Natriumthiosulfat, Alkali-Selenit, Natriumselenit, Ethylendiamin-Tetraessigsäure (EDTA) und/oder DMPS.

## Claims

1. Dental inlay material for tooth cavities which comprises a mercury-binding substance for reducing the mercury concentration in a tooth substance impregnated with mercury from a previously removed amalgam filling, **characterized in that** the mercury-binding substance, in microcrystalline cellulose, comprises at least one of the following materials or consists of one of the following materials: alkali thiosulphate, sodium thiosulphate, alkali selenite, sodium selenite, ethylenediaminetetraacetic acid (EDTA) and/or DMPS.

2. Method for preparing dental inlay material which comprises a mercury-binding substance for reducing the mercury concentration in a tooth substance impregnated with mercury from a previously removed amalgam filling, **characterized by** the use of at least one of the following materials as mercury-binding substance in microcrystalline cellulose: alkali thiosulphate, sodium thiosulphate, alkali selenite, sodium selenite, ethylenediaminetetraacetic acid (EDTA) and/or DMPS.

## Revendications

1. Matériau de comblement des cavités dentaires qui contient une substance qui se lie au mercure pour réduire la concentration en mercure dans une substance dentaire imprégnée de mercure qui provient d'un comblement par un amalgame préalablement enlevé, **caractérisée en ce que** la substance qui se lie au mercure, en cellulose microcristalline, contient au moins l'un des matériaux qui suivent ou est constituée de l'un des matériaux qui suivent : thiosulfate de alcalin, thiosulfate de sodium, sélénite de alcalin, sélénite de sodium, acide éthylènediamine tétraacétique (EDTA) et/ou DMPS.

2. Procédé de préparation d'un matériau de comblement dentaire qui contient une substance qui se lie au mercure pour réduire la concentration en mercure dans une substance dentaire imprégnée de mercure qui provient d'un amalgame de comblement préalablement enlevé, **caractérisé par** l'utilisation dans de la cellulose microcristalline, comme substance qui se lie au mercure d'au moins l'un des matériaux qui suivent : thiosulfate de alcalin, thiosulfate de sodium, sélénite de alcalin, sélénite de sodium, acide éthylènediamine tétraacétique (EDTA) et/ou DMPS.
